# EUROPEAN PATENT APPLICATION

(11) **EP 1 935 372 A1**
(43) Date of publication of application: **25.06.2008**
(21) Application number: 07254873.8
(22) Date of filing: 14.12.2007
(51) Int. Cl.: A61F 2/01, A61M 25/09

(54) **Embolic protection device with distal tubular member for improved torque response**

(30) Priority: 19.12.2006 US 612902
(71) Applicant: Cordis Corporation, Miami Lakes, FL 33014 (US)
(72) Inventor: Wijeratne, Lalith Hiran, Cooper City FL 33328-5168 (US)
(74) Representative: Belcher, Simon James

(57) **Abstract**

An embolic protection device (100) provides a filter (120) for removing emboli from a fluid flowing in a vessel of a patient. The filter is mounted on an elongated tubular member (110) that extends along a substantial distal portion of a core guidewire (102) used to place the filter in the appropriate position within the vessel of the patient. The tubular member (110) provides a way to permit rotation of the core guidewire independent of rotation of the filter while also reducing the friction between the core guidewire (102) and the delivery sheath. Improved torque response result. The tubular member (110) may be one or more pieces that may be either coil wire tube or polymeric tubing.

## Description

The present invention relates to an embolic protection device for use in percutaneous procedures and, more particularly, to an improved embolic protection device having a filter mounted on a rotatable tubular member for filtering bodily fluids flowing through a vessel of a patient.

Various surgical and intravenous treatments have been developed for treating and/or removing obstructions such as plaque in stenosed regions of vessels of a patient. In balloon angioplasty, a balloon placed on the distal end of a catheter is inflated, usually with a fluid, in order to dilate the stenosed region of the vessel thereby improving fluid flow. In addition a stent may be placed in the stenosed region of the vessel in order to reduce the occurrence and severity of restenosis of the vessel. In an atherectomy or thrombectomy, a rotating blade or other similar cutting device can be used to remove plaque in a stenosed region. Surgery may also be used to remove plaque and improve fluid flow in the vessel. All of these treatments result in the production of small particles of plaque or other materials resulting in emboli that can travel in the direction of fluid flow in the vessel and block smaller vessels downstream of the stenosis resulting in stroke, tissue damage or other problems.

In order to prevent such an occurrence various embolic protection devices have been suggested that place a filter distal to the stenosed region before and/or during the angioplasty, stenting, atherectomy or other emboli producing procedure. One type of filter is a woven wire mesh filter. Another type of filter is a polymeric material with filter pores placed on or around a filter frame often made of a self-expanding shape-memory metal such as a nickel-titanium alloy known as nitinol. These filter elements are mounted on the distal end of a guidewire or other elongated member which is used to place the filter into the vessel of the body through an external opening in the body containing the vessel. In some systems a guidewire is first inserted into the lumen of the vessel and is directed past a lesion or other are of interest. After insertion of the guidewire a filter element is pushed along the wire past the lesion or other area of interest where it is deployed. For example, US- 6,179,859 issued to Bates et al. and entitled "Emboli Filtration System and Method of Use" describes a filter that is restrained in a delivery sheath until it is placed in the vessel.

In another type of embolic protection system the guidewire and filter element are simultaneously inserted into the lumen of the vessel and the filter element is directed past the lesion or other area of interest where it is deployed. For example, in US-6,468,291 issued to Bates et al. and entitled "Emboli Filtration System Having Integral Strut Arrangement and Methods of Use" such an embolic protection device is disclosed.

Filter elements are usually mounted on one or more collars either at the distal end, the proximal end or both. These collars are then either fixed to the guidewire or are allowed to travel between one or more stops placed on the guidewire to allow longitudinal movement of the filter basket in relation to the guidewire. The proximal and distal collars of the filter basket may be allowed to rotate on the guidewire to provide rotational movement of the basket relative to the guidewire.

In such embolic protection devices when the guidewire, particularly a guidewire with an embolic protection device, is tracking through a tortuous artery, the guidewire and/or the delivery sheath restraining the embolic protection device is pushed against the vessel wall causing friction between the vessel wall and the device. If a full-length delivery sheath is used, the delivery sheath pushes against the vessel wall which would cause friction between the delivery sheath and the internal guidewire. This friction reduces the torque response of the guidewire. The torque response is defined here as the number of degrees rotated on the proximal end of the wire and the response on the distal end of the wire measured by the number of degrees the distal end of the wire rotates. If this friction were reduced, then the response of the torque response would be improved. Therefore, it would be desirable to have an embolic protection device that would enable the user to accurately navigate the embolic protection device through tortuous arteries and other vessels and provide improved torque response.

It is also desirable to provide an embolic protection device that allows the guidewire to be rotated independent of the basket. Compared with the distal end of the typical guidewire which is approximately 0.37mm (1.1Fr) in size, the profile of the basket is relatively much larger at around 1 - 1.33 mm (3-4Fr) for a 6.0mm basket. When the proximal end of the guidewire is rotated, it takes a relatively large amount of energy to rotate the distal end of the basket. This results in a delay in the response of the distal end when the torque is applied on the proximal end, which is undesirable. In some instances, the distal end does not rotate when the proximal end is rotated thereby storing the energy over the length of the wire. As additional torque is applied, the energy exceeds the frictional forces necessary to start rotating the basket at which time the basket/guide wire "whips" (defined here as uncontrolled rotation of the distal end of the basket/guide wire). This again is undesirable since the physician is unable to control the location of the guide wire tip to guide the tip of the guidewire into a vessel. By having the guide wire move independent of the basket, the phenomenon of guide wire tip "whip" can be significantly reduced.

The present invention generally relates to an embolic protection device for capture and removal of emboli from a fluid flowing in a vessel. More particularly, the invention relates to the distal end of the guidewire on which the filter element of such a device is placed. In the present invention a portion of the distal section of the central guidewire or guidewire core is covered by a tubular member that is free to rotate around the guidewire core. The tubular member may be a coil wire tube made of multiple strands of wire that is wound to form a tubular section. By putting such a tubular member or "sleeve" on the guidewire core that is not attached to the guidewire core itself, the sleeve would push against the tortuous vessel wall while letting the wire rotate freely inside the tubular member.

The present invention results in increased torque response of the guidewire and an improved ability to navigate the guidewire through tortuous vessels. Independent longitudinal movement of the basket with respect to the guidewire means that small movements of the guidewire will not result in the displacement of the basket. The distal section of the wire is well supported by the tubular member thereby preventing kinking of the wire. The tubular member gives more stiffness compared with a polymeric sleeve but yet flexible to track through tortuous vessels and provides additional support to reduce the occurrence of wire prolapse when tracking a stent deployment system over the guidewire.

The length of the tubular member should be substantially the same as the length of the flexible distal section of the guidewire core. Not only is the filter basket independent of the guidewire, but also a substantial portion of the distal end of the device including the filter basket is independent of the guidewire core.

Thus, the present invention is directed to an embolic protection device for filtering emboli in a fluid stream of a vessel of a patient comprising a guidewire core having a proximal end and a distal end and a distal portion extending a first distance from a point near the distal end, a tubular member rotatably mounted about substantially all of the distal portion of the guidewire core, said tubular member having a distal end and a proximal end and a filter fixedly mounted near the distal end of the tubular member. A tubular member is mounted along the first distance of the guidewire core, which extends approximately 10-40 centimeters for a 190 centimeter guidewire. More preferably the length of the tubular member at the distal portion should be between 15 and 35 centimeters for a 190 centimeter guidewire.

The tubular member can be a coil wire tube or a polymeric tube. The embolic protection device may have a filter with a proximal collar and a distal collar to which one or more proximal and distal strut are attached and the tubular member terminates under the proximate collar of the filter.

Alternatively, a second tubular member could extend from the proximal collar of the filter to distal collar of the filter. The filter can be of many types include a filter membrane on a filter frame or a wire mesh filter. The filter membrane could be polymeric, thin film or any other known type of filtering material. The filter may be self-actuating or actuated by a number of means including, but not limited to, mechanical compression, inflation members.

Embodiments of the invention will now be described by way of example with reference to the accompanying drawings, in which:
FIG. 1 is an elevational view of a first embodiment of an embolic protection device in accordance with the present invention.
FIG. 2A is a perspective view of the embolic protection device of FIG. 1 in accordance with the present invention.
FIG. 2B is a sectional perspective view of the embolic protection device of FIG. 1.
FIG. 2C is a sectional perspective view of the distal end of the embolic protection device of FIG. 1.
FIG. 2D is a sectional perspective view of the proximal end of the embolic protection device of FIG. 1.
FIG. 3A is a perspective view of another embodiment of an embolic protection device in accordance with the present invention.
FIG. 3B is a sectional view of the embolic protection device of FIG. 3A.
FIG. 3C is a sectional view of the distal end of the embolic protection device of FIG. 3A.
FIG. 3D is a sectional view of the proximal end of the embolic protection device of FIG. 3A.
FIG. 3E is a perspective view of the filter basket portion of the embolic protection device of FIG. 3A.
FIG. 4A is a perspective view of a further embodiment of an embolic protection device in accordance with the present invention.
FIG. 4B is a sectional perspective view of the embolic protection device of FIG. 4A.
FIG. 4C is a sectional perspective view of the distal end of the embolic protection device of FIG. 4A.
FIG. 4D is a sectional perspective view of the proximal end of the embolic protection device of FIG. 4A.
FIG. 5 is a cross-sectional view of an embodiment of a filter system in accordance with the present invention.

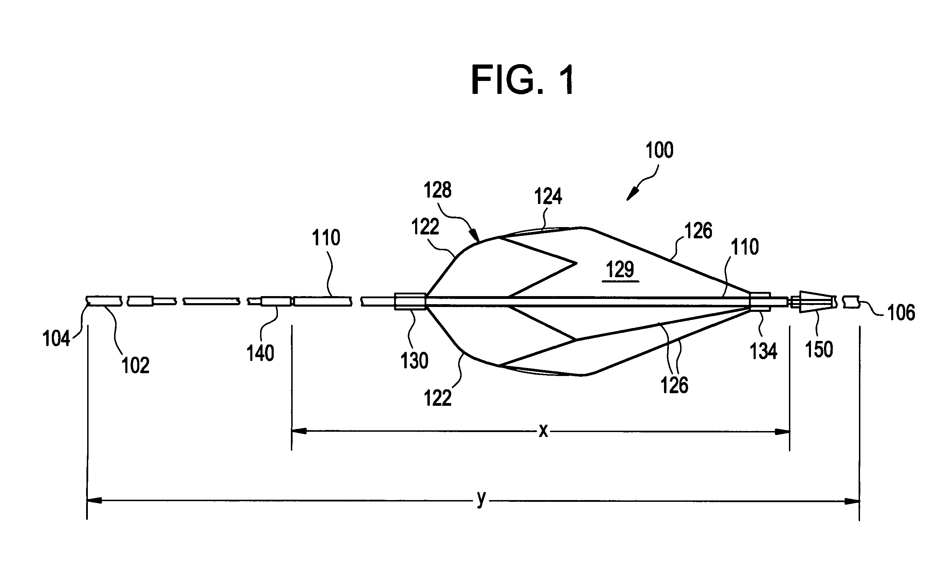

Referring to FIG. 1, embolic protection device **100** comprises a guidewire core **102** having a proximal end **104** and a distal end **106.** Guidewire core **102** is of sufficient length to enable the user of the embolic protection device to route the device to a position in the lumen of a vessel where embolic protection is desired, such as distal to an area of plaque for which an angioplasty procedure is to be used to open the vessel. Typical lengths, y, of the guidewire core 102 are between 150 to 200 centimeter for rapid-exchange systems and between 280 and 330 cm over-the-wire systems but other lengths could be used depending on the application. Guidewire core **102** is metal, preferably stainless steel or another type of non-reactive metal or metal alloy with or without a lubricous coating. The types of lubricious coatings that can be applied to guidewire core **102** include Polytetrafluoroethylene ("PTFE") coatings, silicone coatings and hydrophilic coatings.

A tubular member **110** is placed over a portion of guidewire core **102** near its distal end **106.** Tubular member **110** is a polymeric tube or coil wire tube. In a preferred embodiment a coil wire tube from Asahi Intec Co., Ltd. is used. Tubular member **110** has a diameter slightly larger than the diameter of the core guidewire **102** at the distal end. The tolerance between the guidewire core **102** and the tubular member **110** is preferably approximately 0.025 mm (0.001 inch) but could be 0.013 mm (0.0005 inch) or greater. The tubular member **110** can also be coated with a lubricious coating, such as PTFE, internally and/or externally if desired.

For a tubular member **110** that is a coil wire tube the ends are preferably soldered to prevent the wire from unraveling. Also, the ends of the tubing can be ground down a small amount if necessary to approximately 50% of the original wall thickness.

Tubular member **110** may also be composed of a polymer polymeric blend or a polymer fabric . This would give the same torque characteristics, but would results in less stiffness of the distal end. Polymers that could be used for tubular member **110** include the polymer sold under the trade name PEEK ™ available from Victrex plc, polyamide, polyurethane, nylon PTFE and polyethylene, among others. VICTREX PEEK polymer, is a repeat unit that comprises of oxy-1, 4-phenylenoeoxy-1, 4-phenylene-carbonyl-1, 4-phenylene. PEEK is a linear aromatic polymer that is semicrystalline.

Tubular member **110** floats freely on the core guidewire **102** and is not attached to the core guidewire at any point along its length. Tubular member **110** is of a length, x, that is between approximately 10 and 40 centimeters for a rapid exchange system that would be between 150 to 200 centimeters in length. Preferably tubular member should have a length between 15 and 35 centimeters for such a rapid exchange system. Tubular member **110** should have a length between approximately 15 and 35 centimeters for an over-the-wire system that would be between approximately 280 and 330 centimeters in length. The important attribute of the present invention is that the tubular member **110** extend not only under the proximal end of filter **120** but that it extends a substantial distance proximal the filter **120.** Preferably tubular member **110** extends at least approximately 15 centimeters along the guidewire core **102** in the proximal direction. Too long of a tubular member will unnecessarily add to the cost of the device without providing significant added benefit. Additionally, a significantly longer tubular member 110 will result in reduced pushability.

A longitudinal motion limiter **140** is a polymer sleeve or other motion limiting device attached to the core guidewire **102** to prevent migration of the tubular member **110** toward the proximal end of the core guidewire **102.** Attachment of the polymer sleeve may be by any appropriate bonding means. Alternatively, core guidewire may be tapered at this point to transition to a larger diameter at the proximal end of the distal portion in order to prevent such migration.

Filter 120 in FIG. 1 is depicted as having a filter frame **128** having a plurality of proximal struts **122** connected at the proximal end to proximal collar **130** and at the distal end to apposition member **124.** The number of proximal struts is not critical to the invention, but preferably there are at least two proximal struts and more preferably four proximal struts. Apposition member **124** is used to provide proper apposition of the filter **120** against the wall of the vessel. Apposition member **124** is also connected to a plurality of distal struts **126** which are connected to a distal collar **130.** Filter frame **128** may be constructed by cutting a single tube of material or may be constructed from a number of different combinations of proximal and distal struts and an apposition member, proximal collar and distal collar which may be interconnected by a number of known means such as welding, brazing or chemical bonding. Filter frame **128** may be made of any number of metal alloys but is preferably made from a "shape memory" metal such as nitinol.

Filter membrane **129** is attached to filter frame **128** and may be comprised of a polymeric blend or may also be made of a thin film of nitinol or other similar metal exhibiting memory characteristics. If filter membrane **129** is a polymeric membrane it can be bonded to filter frame **128** or can be molded around the filter frame **128** as part of the manufacturing process. Filter membrane **129** contains a plurality of holes which are sized to provide a path for fluid flow but are not large enough to permit emboli to pass. The preferred hole size is 0.1mm (0.00394 inch) and there are preferably approximately 3100 holes per square centimeter. Distal tip **150** is a tapered polymeric sleeve or a tapered coil that provides an atraumatic transition between the distal end **106** of the core guidewire **104** and the filter **120.**

Other types of filter elements can be substitute for the filter **120** of FIG. 1 of the present invention. For example, filter **120** could be a wire mesh filter made of a woven wire fabric wherein the interstices between the metal wires provides the proper filtering function or a thin film nitinol mesh. Additionally filter **120** and/or filter membrane **129** could be comprised of woven polymeric threads. Filter **120** may be a self-actuating filter, i.e., one that expands to oppose the vessel wall upon removal from a restraining sheath or other restraining mechanism or the filter may be one that requires actuation such as through mechanical compression or inflation of an actuation balloon. A self-actuating filter is preferred because lack of an actuator results in a lower profile embolic protection device. Additionally, filter **120** could be a mechanically actuated filter requiring one or more wires, rods or other means of mechanical actuation. Other means of filter actuation are also possible.

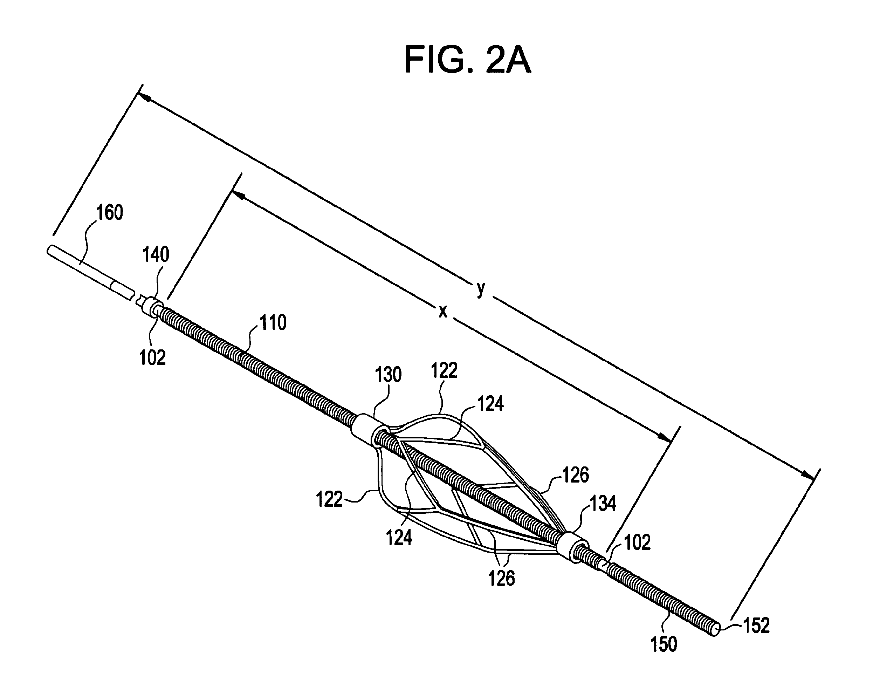

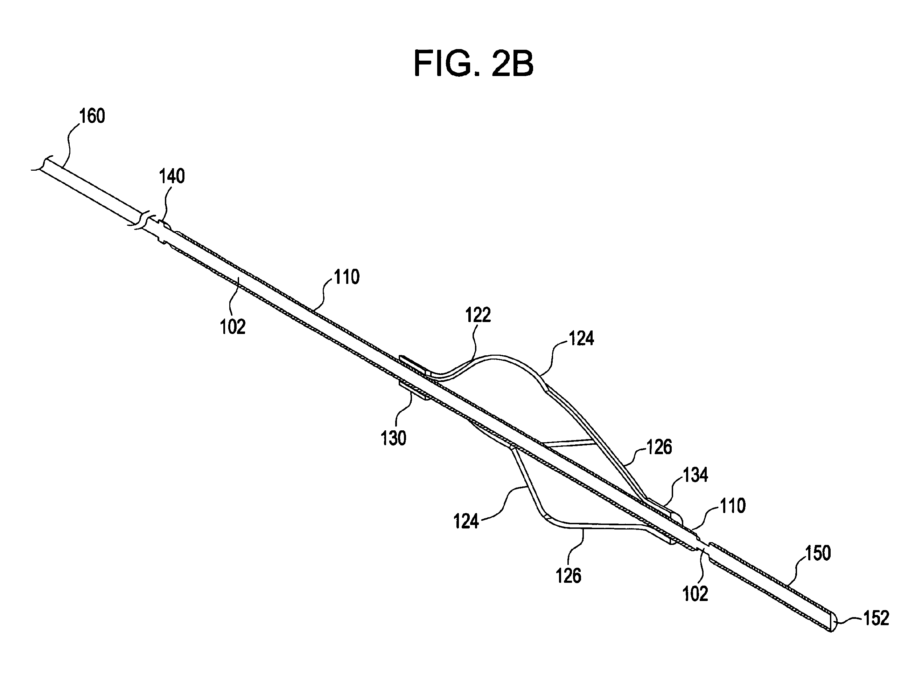

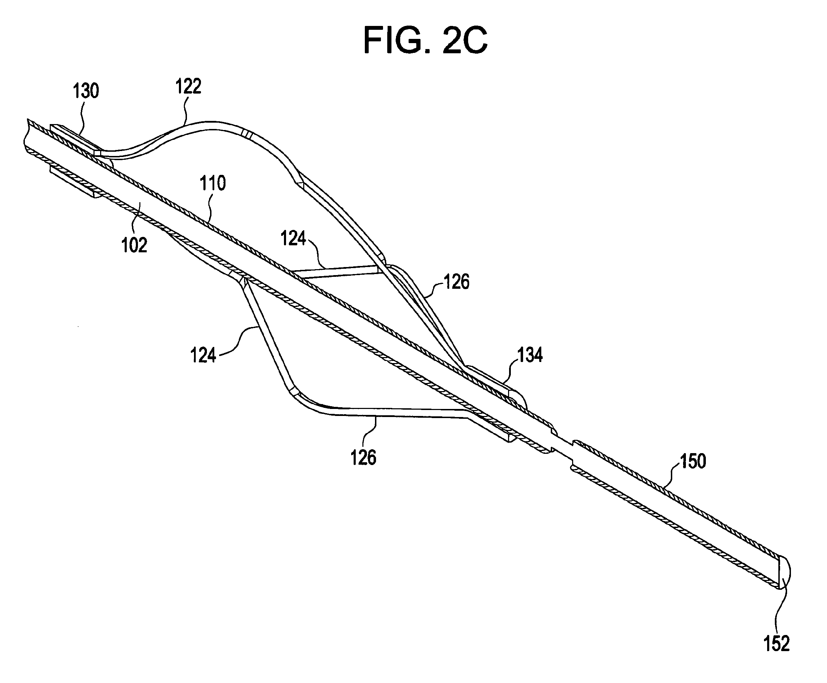

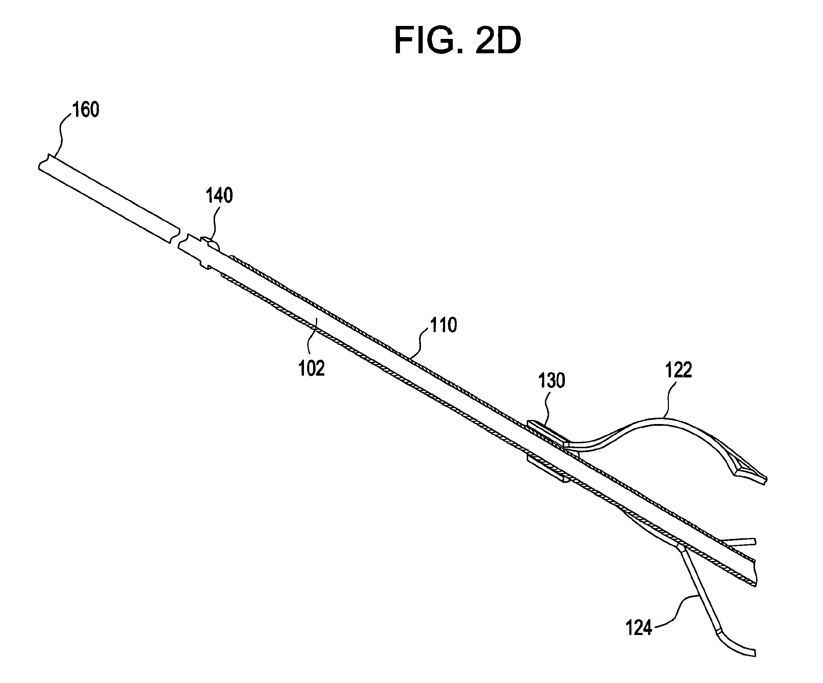

FIGS. 2A-D depict the embolic protection device of FIG. 1 in perspective and sectional perspective views including the additional feature of a motion limiter **140** which limits the axial movement of the filter assembly relative to the guidewire. The sectional view of FIG. 2B shows that guidewire core **102** extends the length of the embolic protection device from motion limiter **140** to the distal end covered by distal tip **150** with an atraumatic bullet nose **152.** Tubular member **110** extends from a substantial distance proximally of the filter basket **128** under proximal collar **130** and past distal collar **134.** Proximal collar **130** is fixedly mounted on the tubular member **110** using one of many known means of fixation such as bonding, soldering, brazing, or epoxying. Distal collar **134** is free floating on the tubular member **110.** In an alternative configuration proximal collar **130** and distal collar **134** could be rotatably mounted on tubular member **110** through the use of one or more longitudinal motion limiters (not shown) and or bearing surfaces (not shown) that would provide a second level of rotational freedom between the guidewire core **102** and the filter **120.**

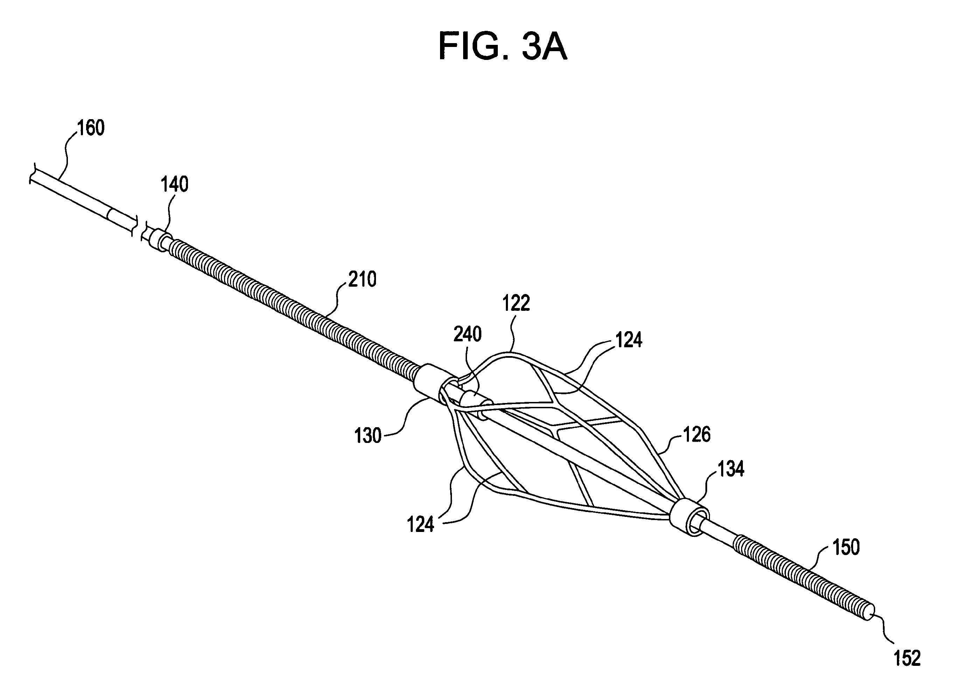

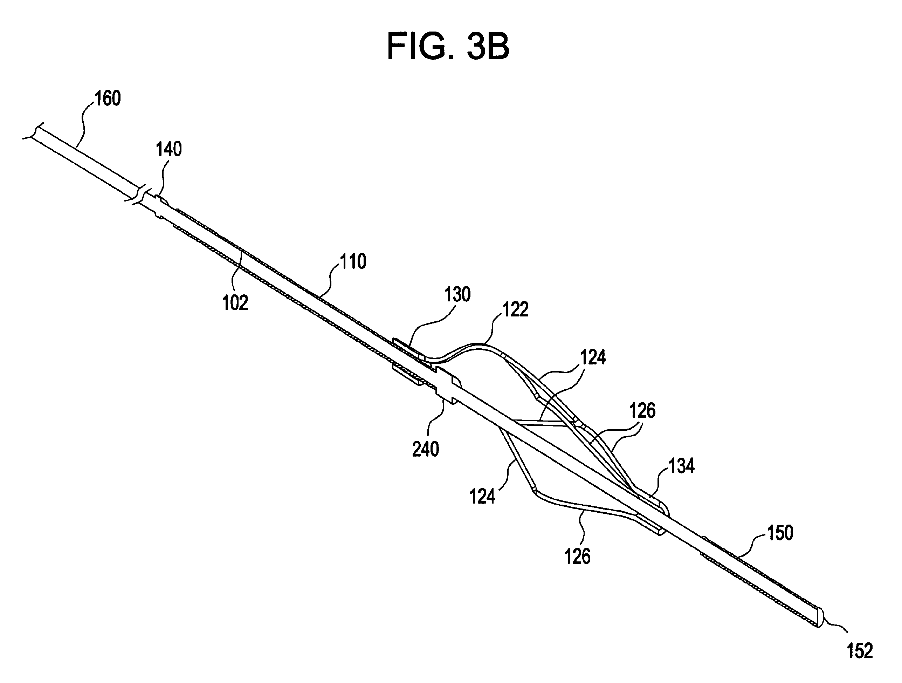

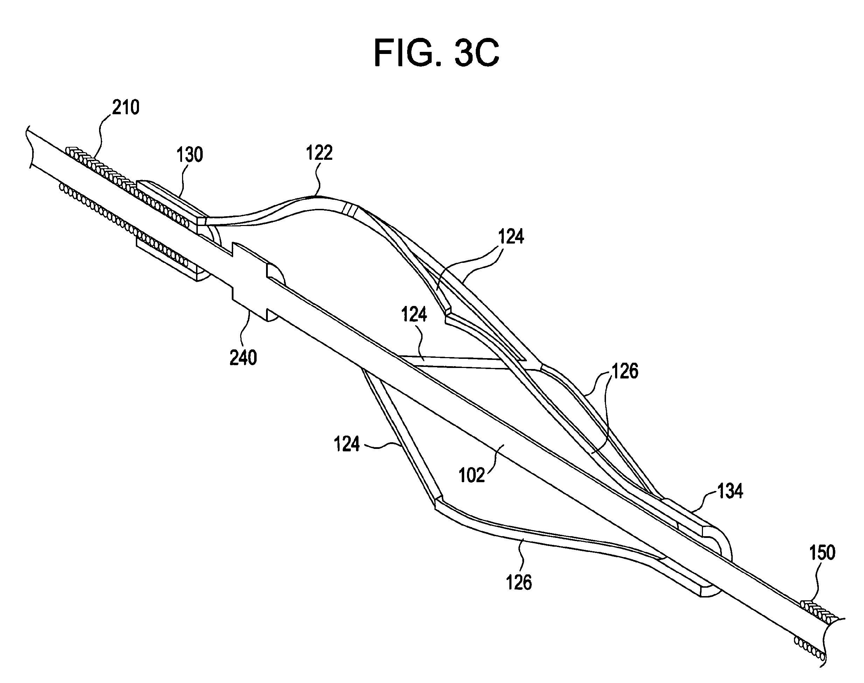

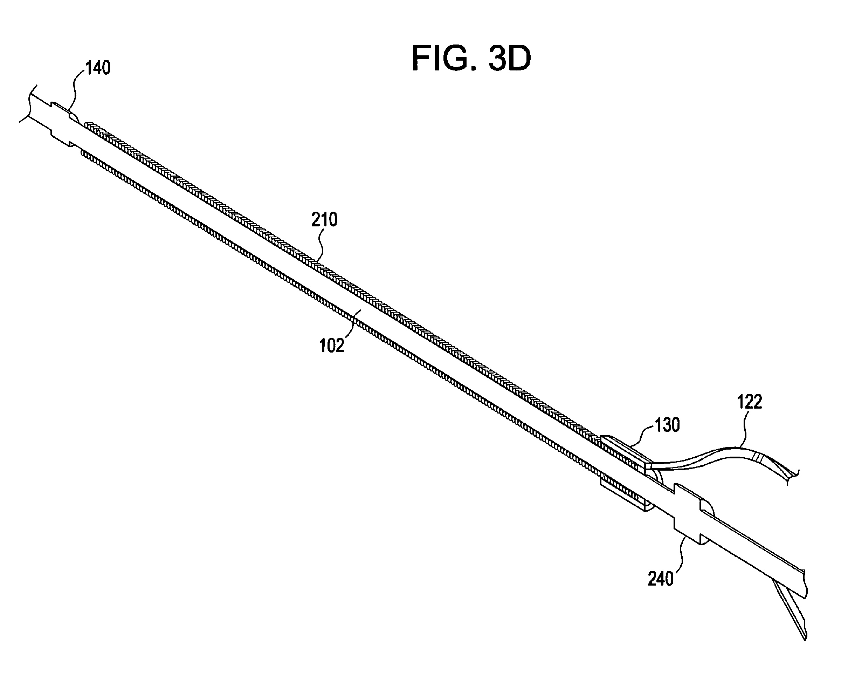

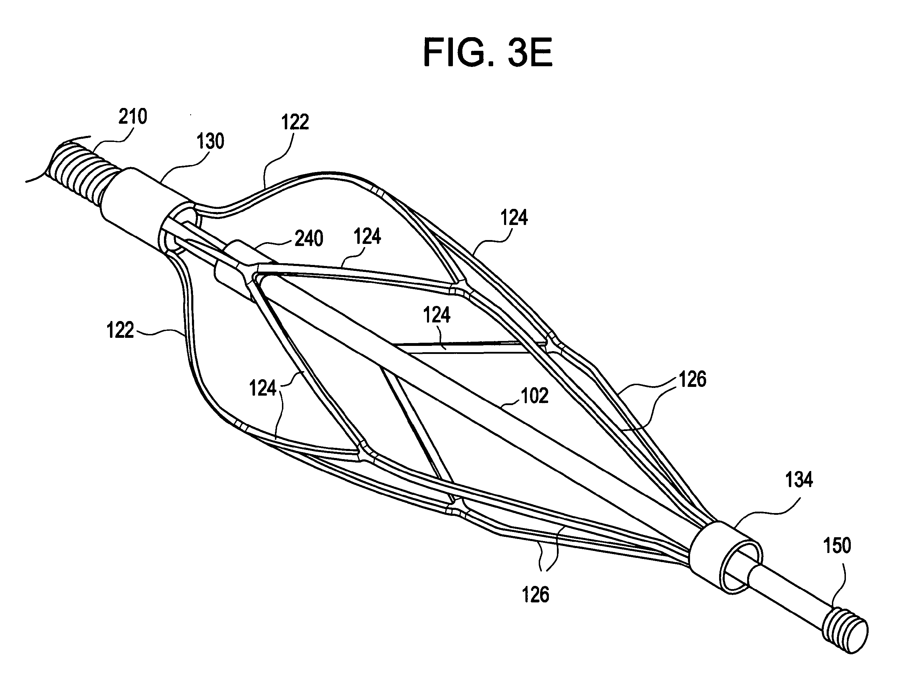

FIGS. 3A-E depict another embodiment of the present invention in which the tubular member **210** extends from the same distance at the proximal end of the distal portion of the core guidewire **102** to the proximal collar **130** of the filter **120.** This reduces the profile of the filter **120** further. A thin walled polyimide sleeve (not shown) may be placed around the guidewire core 102 so that the filter membrane 129 will not grab on the guidewire core **102** when the filter is in a collapsed configuration. When filter frame **128** is made from a single tube of material such as when it is cut from a nitinol tube, the ID of each of the proximal collar **130** and the distal collar **134** is fixed. Since the tubular member **210** does not extend under the distal collar 134 of the filter the ID must be reduced so that it rotates around the guidewire core **102.** Two possible options to reduce the ID are to place a sleeve (which could be radiopaque) on the ID of the distal collar **134** or to position the guidewire coil such that the guidewire coil resides inside of the distal leg when the filter is in an expanded state. Clearance between the guidewire core **102** and the distal collar **134** should be minimized in order to prevent emboli from escaping the filter **120.** The embodiment in FIGS. 3A-E also include a second longitudinal motion limiter **240** which limits the movement of the filter assembly relative to the guidewire.

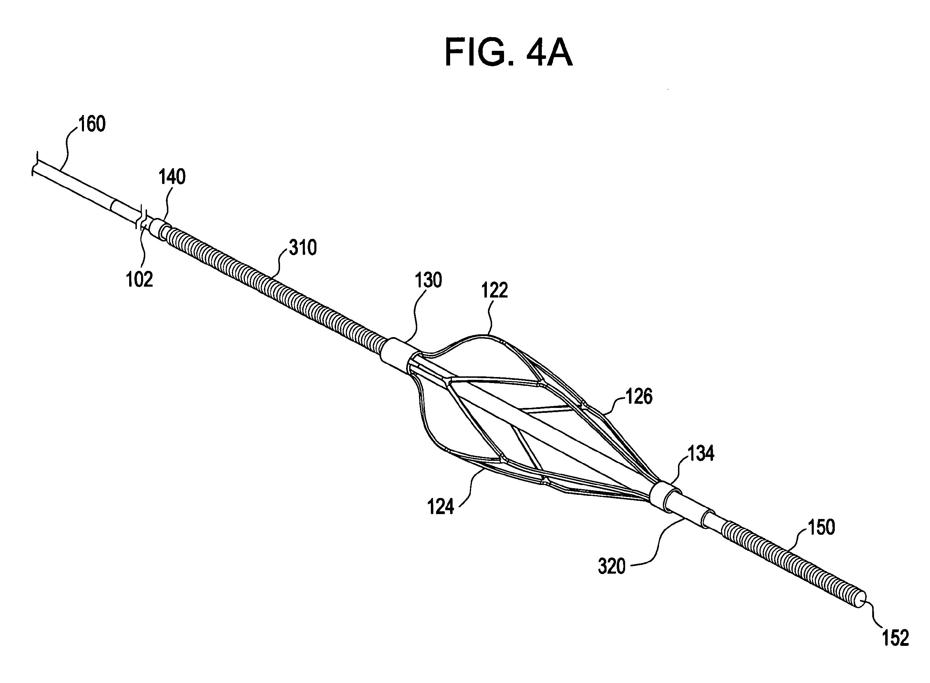

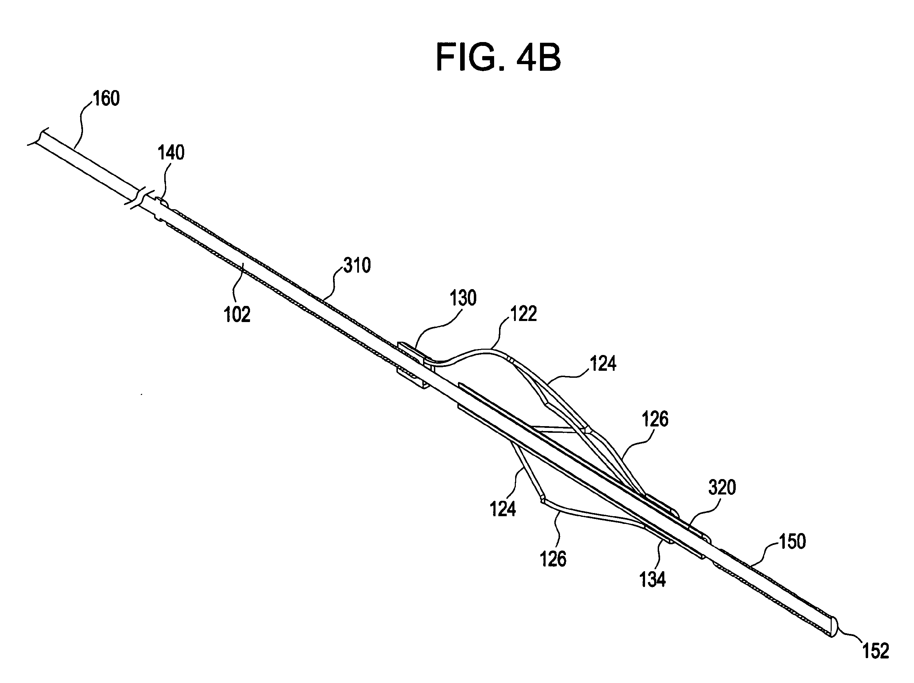

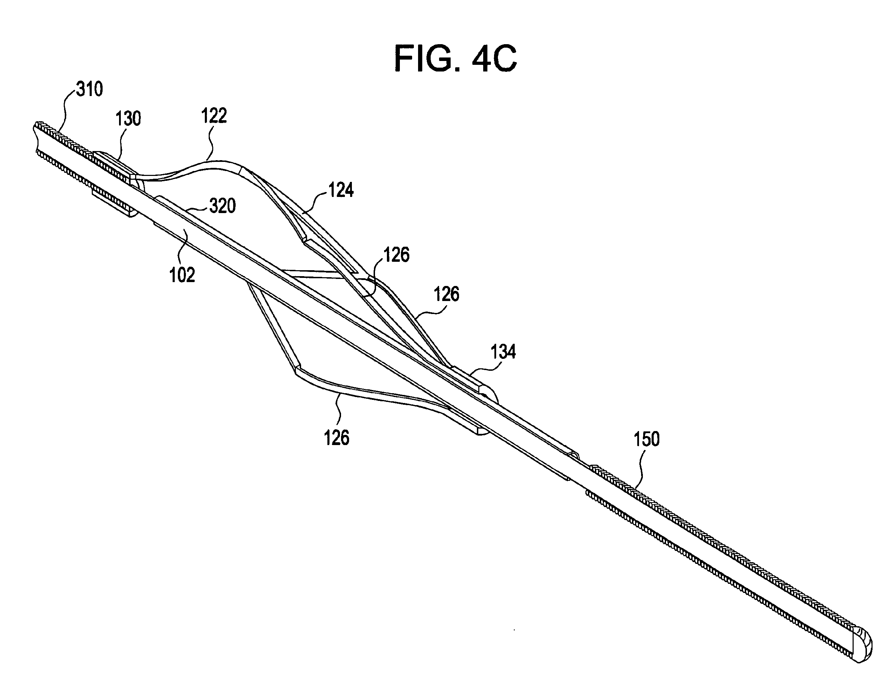

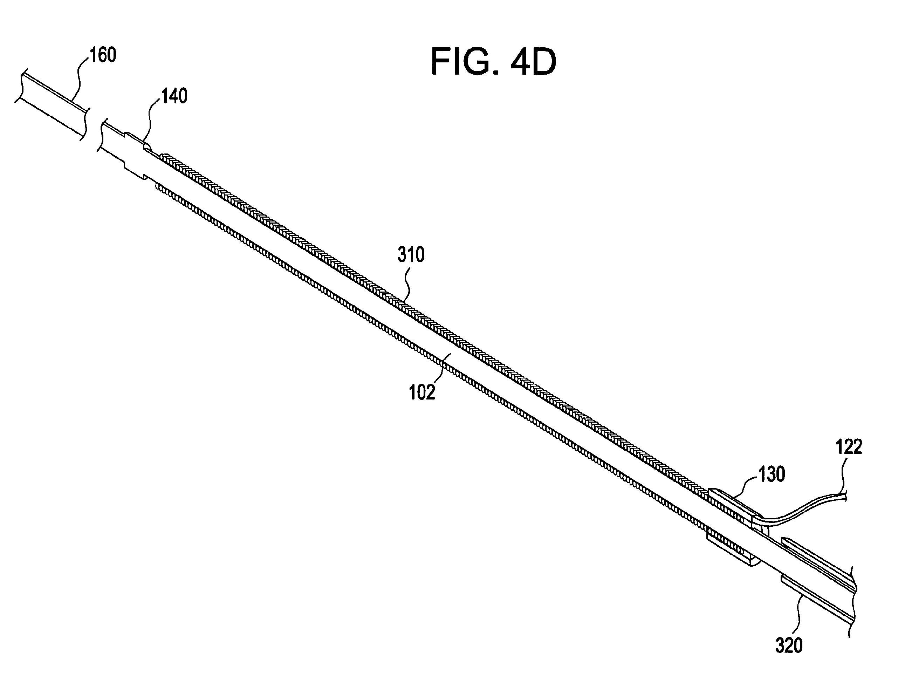

An additional alternative embodiment is depicted in FIGS. 4A-4D. A first tubular member **310** extends from the proximal end of the distal portion of the core guidewire (as discussed above with regard to FIG. 1) to the proximal collar **130** of filter **120.** A second tubular member **320** extends from proximal collar **130** to distal collar **134.** Tubular member **320** may be connected to tubular member **310** or may rotate independently. Tubular member **320** may extend beyond the distal end of the basket giving a flexible transition between the filter **120** and the distal tip **150.**

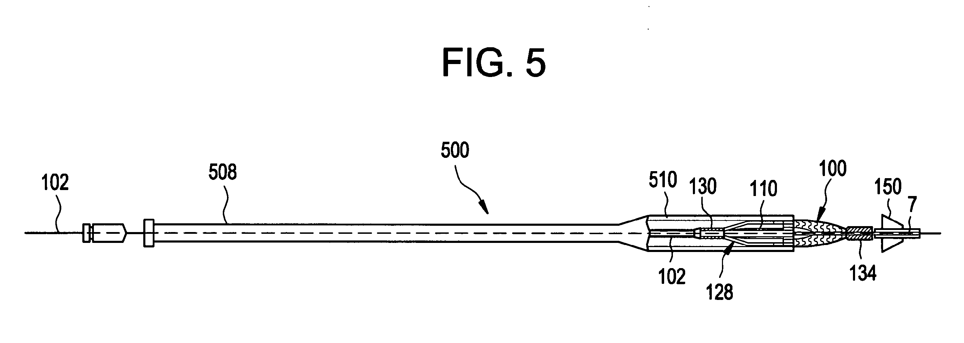

FIG. 5 depicts an embodiment of an embolic protection filter system in accordance with the present invention. In FIG. 5 the embolic protection device 100 is placed within a deployment and delivery system 500 that comprises a distal portion 510 coaxially disposed around the core guidewire 102 and the filter 120 with filter frame 128, proximal collar 130, distal collar 134, and tubular member 110. Narrower proximal portion 508 of delivery system 500 is disposed around core guidewire 102. In use the deployment and delivery system 500 with an embolic protection device 100 contained therein is placed through an incision in the patient into the lumen of a vessel of the patient. Once the distal end of the embolic protection filter system is placed distally of the region of interest and/or treatment such as a lesion, the filter is uncovered and the filter basket radially expands to oppose the lumen of the vessel of the patient. Upon completion of the procedure at the treatment site, the filter is collapsed using a retrieval catheter (not shown) as is known in the art. The embolic protection filter system is then removed from the vessel of the patient.

## Claims

1. An embolic protection device for filtering emboli in a fluid stream of a vessel of a patient comprising:
a guidewire core having a proximal end and a distal end and a distal portion extending a first distance from the distal end;
a tubular member rotatably mounted about substantially all of the distal portion of the core guidewire, said tubular member having a distal end and a proximal end; and,
a filter mounted near the distal end of the tubular member.

2. The embolic protection device of claim 1 wherein the length of the tubular member is between approximately 10 and approximately 40 centimeters.

3. The embolic protection device of claim 1 wherein the tubular member extends at least approximately 15 centimeters proximally of the filter.

4. The embolic protection device of claim 1 wherein the filter is fixedly mounted near the distal end of the tubular member.

5. The embolic protection device of claim 1 wherein the filter is rotatably mounted near the distal end of the tubular member.

6. The embolic protection device of claim 1 wherein the tubular member is a coil wire tube.

7. The embolic protection device of claim 1 wherein the tubular member is a polymeric tube.

8. The embolic protection device of claim 1 wherein the filter has a proximal collar and a distal collar to which one or more proximal and distal struts are attached and the tubular member terminates under the proximal collar of the filter.

9. The embolic protection device of claim 8 further comprising a second tubular member extending from the proximal collar of the filter to distal collar of the filter.

10. The embolic protection device of claim 9 wherein the second tubular member is a polymeric tube.

11. The embolic protection device of claim 9 wherein the second tubular member is a coil wire tube.

12. The embolic protection device of claim 9 wherein the second tubular member is fixedly connected to the tubular member.

13. The embolic protection device of claim 1 wherein the filter is a wire mesh filter.

14. The embolic protection device of claim 1 wherein the filter comprises a metal frame having a filter membrane attached thereto.

15. The embolic protection device of claim 14 wherein the filter membrane is a polymeric membrane having a plurality of holes.

16. The embolic protection device of claim 14 wherein the filter membrane is a sheet of nitinol having a plurality of holes.

17. The embolic protection device of claim 15 wherein the holes in the polymeric membrane are approximately 0.1 mm in diameter.

18. The embolic protection device of claim 1 wherein the tubular member is coated with a lubricious coating.

19. The embolic protection device of claim 15 or 16 wherein the filter membrane contains approximately 3100 holes per square centimeter.
